# EUROPÄISCHE PATENTSCHRIFT

(11) **EP 2 908 895 B1**
(45) Veröffentlichungstag und Bekanntmachung des Hinweises auf die Patenterteilung: **11.07.2018**
(21) Anmeldenummer: 13776998.0
(22) Anmeldetag: 08.10.2013
(51) Int. Cl.: A61M 16/08, A61M 16/04, A61M 16/10, A61M 16/20, A61F 2/20

(54) **SPRECHVENTIL FÜR TRACHEOSTOMIEKANÜLE**
SPEAKING VALVE FOR TRACHEOSTOMY CANNULA
VALVE DE PHONATION DESTINÉE À UNE CANULE DE TRACHÉOTOMIE

(30) Priorität: 17.10.2012 DE 102012109916
(43) Veröffentlichungstag der Anmeldung: 26.08.2015
(73) Patentinhaber: Tracoe Medical GmbH, 55268 Nieder-Olm (DE)
(72) Erfinder: SCHNELL, Ralf, 63500 Seligenstadt (DE)
(74) Vertreter: WSL Patentanwälte Partnerschaft mbB
(86) Internationale Anmeldenummer: PCT/EP2013/070921
(87) Internationale Veröffentlichungsnummer: WO 2014/060242

(56) Entgegenhaltungen:
- EP-A1- 2 236 165
- WO-A1-2011/062533
- DE-U1-202005 006 605
- DE-U1-202012 001 825
- GB-A- 2 313 317
- US-A- 4 759 356
- US-A- 5 738 095
- US-A1- 2012 097 170
- US-B1- 6 386 200
- US-B1- 6 802 316

## Beschreibung

Die Erfindung betrifft ein Sprechventil zum Aufsetzen auf eine Tracheostomiekanüle, das eine Ventileinheit und eine Aufsteckeinheit umfasst, wobei die Aufsteckeinheit ein rohrförmiges Gehäuse aufweist, mit einem ersten und einem zweiten Ende und einer vom ersten zum zweiten Ende verlaufenden Längsachse, wobei das erste Ende der Aufsteckeinheit mit einem Konnektor an der Tracheostomiekanüle verbindbar ist und die Ventileinheit an dem zweiten Ende der Aufsteckeinheit angeordnet ist und wobei die Ventileinheit und die Aufsteckeinheit beweglich miteinander verbunden sind, sowie eine mit einem solchen Sprechventil ausgestattete Tracheostomiekanüle.

Bei Patienten, deren Atmung durch ein Tracheostoma, in das eine Tracheostomiekanüle eingebracht ist, erleichtert wird, dringt Luft stromabwärts des Kehlkopfs in die unteren Atemwege ein und auch wieder aus. Ein Austreten der Luft durch das Tracheostoma bzw. die Tracheostomiekanüle kann durch einfaches Blockieren des Kanülenrohrs z.B. mit dem Finger verhindert werden, wodurch die Luft dann bei der Ausatmung über die natürlichen Atemwege, d.h. den Kehlkopf und den Mund bzw. die Nase geleitet wird, und die Stimmbänder für die Tonerzeugung in Schwingung gesetzt werden können.

Um tracheostomierten Patienten das Sprechen zu erleichtern, sind Ventile bekannt, die zur Anbringung an eine Tracheostomiekanüle vorgesehen sind und so gestaltet sind, dass sie beim Einatmen öffnen, so dass Luft durch die Tracheostomiekanüle einströmen kann und beim Ausatmen schließen, so dass die Luft über die natürlichen Atemwege geleitet wird, ohne die Tracheostomiekanüle manuell verschließen zu müssen. Bei der Verwendung derartiger Ventile wird nicht nur das Sprechen erleichtert, sondern zusätzlich durch Leiten von ausgeatmeter Luft über die natürlichen Atemwege auch ein therapeutischer Zweck erzielt, nämlich u. a. das Trainieren der Schluckreflexe.

Die Verwendung eines Sprechventils kann jedoch nach längerer Zeit für einen Patienten sehr anstrengend und unkomfortabel sein. Dies liegt einerseits daran, dass der Kraftaufwand, den der Patient zum Ausatmen über die natürlichen Atemwege aufbringen muss, ermüdend sein kann.

Dieser erhöhte Kraftaufwand ist unter anderem dadurch bedingt, dass die für die eigenständige Atmung notwendige Muskulatur bei tracheostomierten Patienten nach einiger Zeit nachlässt und weniger trainiert ist. Andererseits kann die Ventilfunktion dazu führen, dass mehr Luft eingeatmet wird als ausgeatmet werden kann, das sogenannte "Air-Trapping". Es entsteht dadurch ein für den Patienten unangenehmer Überdruck in den unteren Atemwegen. Auch das Einatmen kann durch den Aufsatz eines Sprechventils erschwert sein. WO2011062533 beschreibt eine Sprechventil zum Einatmen und Sprechen. Um dieses Problem zu beheben sind Sprechventile bekannt, die aufklappbar sind, um ein Ausatmen durch die Tracheostomiekanüle zu ermöglichen, wenn ein Sprechen und damit das Ausatmen über die natürliche Atemwege nicht gewünscht bzw. nicht erforderlich ist. Bei anderen bekannten Sprechventilen wird das Problem dadurch gelöst, dass zwischen der Tracheostomiekanüle und dem eigentlichen Ventil seitliche Fenster angebracht sind, die sich durch Verschieben eines dazu verschiebbar angeordneten Elements mit Schlitzen öffnen und schließen lassen.
Bei den meisten Sprechventilen sind keine derartigen Vorrichtungen vorgesehen, so dass bei Auftreten der oben geschilderten Probleme das Sprechventil vollständig von der Tracheostomiekanüle entfernt werden muss.
Vor dem Hintergrund dieses Stands der Technik bestand daher die Aufgabe, eine Alternative zu bekannten Sprechventilen bereitzustellen, bei der es trotz Nutzung eines Sprechventils möglich ist, sofern dies gewünscht ist, durch Umgehung der Ventilfunktion durch das Tracheostoma auszuatmen.
Erfindungsgemäß wird diese Aufgabe durch ein Sprechventil der eingangs genannten Art gelöst, dass die Ventileinheit so ausgestaltet und angeordnet ist, dass sie
in einer ersten Position das Gehäuse der Aufsteckeinheit bezüglich einer Strömungsrichtung luftdicht verschließt und
in wenigstens einer zweiten Position einen Luftstrom in das Gehäuse oder aus diesem heraus zulässt,
wobei die Ventileinheit durch eine Bewegung parallel zu der Richtung der Längsachse des rohrförmigen Gehäuses von der ersten in die wenigstens eine zweite Position überführbar ist.
Die erste Position entspricht dabei der bekannten Funktionsweise eines Sprechventils bei welcher nur das Einatmen durch das Ventil möglich ist, während das Ausatmen durch ein Ventilelement verhindert wird und die Atemluft somit über die natürlichen Atemwege entweichen muss. Die zweite Position definiert hingegen ein in beiden Richtungen offenes Sprechventil.

Durch diese Ausgestaltung ist es möglich, ein Sprechventil auf kleinem Raum bereitzustellen, bei dem die Ventilfunktion leicht und ohne schmerzhafte Manipulation an der Tracheostomiekanüle umgangen werden kann. Zudem kann durch Bereitstellen mehrerer "zweiter" Positionen des Ventilabschnitts eine Öffnung zwischen Ventileinheit und Aufsteckeinheit so variiert werden, dass unterschiedliche Strömungswiderstände für das Ausatmen durch das Tracheostoma entstehen. Dadurch können Positionen eingestellt werden, bei denen ein Teil der eingeatmeten Luft durch die oberen Atemwege entweicht und ein Teil durch die Aufsteckeinheit des Sprechventils entweicht.

Für die reguläre Funktion eines so gestalteten Sprechventils ist die Ventileinheit dafür vorgesehen und so aufgebaut, dass Luft durch das Sprechventil in die Tracheostomiekanüle und letztlich in die Lunge gelangen kann, auch wenn sich die Ventileinheit in der ersten Position befindet, wobei in dieser Position, bei der die Ventileinheit luftdicht an dem rohrförmigen Gehäuse der Aufsteckeinheit anliegt, ein Luftdurchlass in die Gegenrichtung (d. h. beim Ausatmen) durch die Ventilwirkung der Ventileinheit unterbunden wird. Die Funktion der Ventileinheit besteht somit darin, einen Luftstrom von außen durch die Ventileinheit in Richtung Aufsteckeinheit zuzulassen und einen Luftstrom in die Gegenrichtung zu unterbinden. Für das Bereitstellen diese Funktion können bekannte Ventilvorrichtungen in der Ventileinheit oder als Ventileinheit verwendet werden.

Vorzugsweise liegen in der ersten Position eine ringförmige, im Wesentlichen senkrecht zur Längsachse oder konisch verlaufende Fläche am zweiten Ende des rohrförmigen Gehäuses und eine ringförmige Stirnfläche der zu der Aufsteckeinheit weisenden Seite der Ventileinheit luftdicht aneinander an

Die Ventileinheit ist in einer Ausführungsform so aufgebaut, dass sie in der ersten Position die kreisförmige Öffnung am zweiten Ende des rohrförmigen Gehäuses vollständig bedeckt und ggf. radial über den Rand dieser Öffnung hinausragt.

Der Begriff rohrförmiges Gehäuse bezeichnet ein im Wesentlichen hohlzylinderförmiges Gehäuse, wobei dessen Innenwand und/oder Außenwand konisch zulaufen kann, insbesondere in Richtung des zweiten Endes. Das rohförmige Gehäuse der Aufsteckeinheit ist an beiden Enden offen. Das rohrförmige Gehäuse weist in bestimmten Ausführungsformen an seiner Außen- und/oder Innenseite abschnittsweise Aufweitungen oder Verjüngungen auf. Durch eine abschnittsweise Aufweitung in der Nähe des zweiten Endes wird zum Beispiel Raum für das Einbringen eines Filters oder anderer Gegenstände bzw. Raum für eine Bewegung eines Bestandteils der Ventileinheit bei Öffnen des Ventils für einen Lufteinstrom in Richtung der Aufsteckeinheit bzw. in die Aufsteckeinheit hinein bereitgestellt.

Die "Längsachse des rohrförmigen Gehäuses", wie hier verwendet, bezeichnet die Achse, die zentral von dem ersten zu dem zweiten Ende des rohrförmigen Gehäuses verläuft, und bezüglich der das rohrförmige Gehäuse eine Rotationssymmetrie aufweist, wobei der Begriff rohrförmiges Gehäuse auch Gehäuse umfasst, deren Querschnitt im Wesentlichen mehreckig, beispielsweise vier-, fünf-, sechs- oder achteckig ist.

In einer Ausführungsform beträgt die Ausdehnung des rohrförmigen Gehäuses bzw. der Aufsteckeinheit auf der Außenseite zwischen 16 und 26 mm.

In einer Ausführungsform ist die Ventileinheit durch eine Bewegung parallel zu der Richtung der Längsachse des rohrförmigen Gehäuses stufenlos von der ersten in eine zweite Position überführbar und es sind zwischen der ersten und zweiten Position beliebig viele Positionen einstellbar, in denen die Ventileinheit von dem Gehäuse der Aufsteckeinheit beabstandet ist. Dies geschieht vorzugsweise zum Beispiel durch einen Gewindeeingriff zwischen Aufsteckeinheit und Ventileinheit.

Dabei weist in einer Ausführungsform die Aufsteckeinheit ein Außengewinde und die (ebenfalls einen Rohrabschnitt aufweisende) Ventileinheit ein dazu passendes Innengewinde auf oder umgekehrt.

In einer Ausführungsform ist die Ventileinheit zwischen der ersten und zweiten Position bewegbar und es sind verschiedene definierte zweite Positionen einstellbar, in denen die Ventileinheit einen Luftdurchlass zu und von dem Inneren der Aufsteckeinheit mit jeweils unterschiedlichem Strömungsquerschnitt bzw. Strömungswiderstand zulässt.

In einer Ausführungsform ist die Ventileinheit als Ventilkappe mit einem zylindrischen Kappenmantel und einem scheibenförmigen Ventildeckel ausgebildet.

Zweckmäßigerweise weist der Kappenmantel mindestens eine Aussparung auf, die in der ersten Position durch eine an der Umgebung der Aussparung anliegende Wand der Aufsteckeinheit verdeckt und in der zweiten Position durch die Wand der Aufsteckeinheit freigegeben wird Beispielsweise besteht die Aufsteckeinheit im Wesentlichen aus einem rohrförmigen Gehäuse mit Aufsteck- bzw. Verbindungselementen an dem ventilseitigen und dem entgegengesetzten Ende. Die Ventileinheit kann z.B. die Form einer Kappe mit einem zylindrischen Mantel haben, die auf ein Ende der rohrförmigen Aufsteckeinheit aufgesetzt ist, wobei der Innendurchmesser des Kappenmantels im Wesentlichen dem Außendurchmesser der Aufsteckeinheit entspricht und wobei diese beiden Elemente auch durch Gewindeeingriff miteinander verbunden sein können.

Der Deckel der Kappe bildet einen Ventilsitz mit mehreren Durchbrechungen, die von der Innenseite durch eine Ventilmembran abgedeckt sind. Zusätzlich kann auch der zylindrische Kappenmantel in der Nähe des Deckels Aussparungen bzw. Durchbrechungen aufweisen, die in der ersten Position von der Wand der rohrförmigen Aufsteckeinheit verdeckt werden, wobei vorzugsweise der Rand an der ventilseitigen Stirnseite des Aufsteckelementes mit der Innenfläche des Deckels bzw. der Ventileinheit in dichtenden Kontakt tritt. Hierzu kann beispielsweise der Übergang zwischen der zylindrischen Mantelfläche und dem Deckel an der Innenseite der Ventileinheit auch konisch ausgebildet sein, was einen guten Dichteingriff sicherstellt. Alternativ kann aber auch ein äußerer, zylindrischer Endabschnitt der Aufsteckeinheit passend und dicht in dem mindestens eine Aussparung aufweisenden Kappenmantel geführt sein, sodass die Wand der Aufsteckeinheit in der ersten Position die Aussparung bzw. Öffnung im Kappenmantel verdeckt und in einem axial relativ zu der Kappe bzw. dem zylindrischen Abschnitt der Ventileinheit verschobenen Position die Aussparung freigibt, sodass Luft durch die Aussparung und in die stirnseitige Öffnung des Aufsteckelementes einströmen kann.

Hierzu reicht es beispielsweise aus, wenn die Ventileinheit relativ zur Aufsteckeinheit um ein Maß von 2 bis 5, vorzugsweise zwischen 3 und 4 mm axial verschiebbar ist, wobei die erste Position dadurch definiert wird, dass ein stirnseitiges Ende des Aufsteckelementes an dem Deckel des der kappenförmigen Ventileinheit oder an einem konischen Übergang auf der Innenseite zwischen zylindrischem Mantel und Deckel der Ventileinheit anschlägt, während die zweite Position durch einen Abstand des Randes der Aufsteckeinheit von dem Ventildeckel gekennzeichnet ist und dieser Abstand zwischen 2 bis 5, vorzugsweise weniger als 4 und insbesondere etwa 3 mm beträgt.

In einer Ausführungsform ist die Ventileinheit unabhängig von der ersten und der wenigstens einen zweiten Position zu der Aufsteckeinheit auch vollständig von der Aufsteckeinheit abtrennbar. Dies hat den Vorteil, dass die einzelnen Elemente des Sprechventils leichter gereinigt, desinfiziert und (zum Beispiel mit Blick auf einen Feuchtigkeits/Wärmespeicher oder Filter) ausgetauscht werden können.

In einer Ausführungsform weist die Aufsteckeinheit wenigstens ein erstes Verbindungselement auf und die Ventileinheit wenigstens ein zweites Verbindungselement, wobei das wenigstens eine erste und wenigstens eine zweite Verbindungselement so gestaltet sind, dass sie je eine Aussparung und einen dazu korrespondierenden Vorsprung bilden, und die Aufsteckeinheit und die Ventileinheit über die miteinander in Eingriff stehenden ersten und zweiten Verbindungselemente beweglich verbunden sind. Für die Ausgestaltung der Verbindungselemente der Aufsteckeinheit bzw. der Ventileinheit sind derartige Verbindungselemente vorteilhaft, da sie eine Bewegung zwischen einer ersten Position und wenigstens einer zweiten Position gestatten.

Bei der zuvor bereits beschriebenen Variante werden die Verbindungselemente einerseits durch das rohrförmige Gehäuse der Aufsteckeinheit und andererseits durch den Zylindermantel eines Kappenförmigen Ventilelementes gebildet. In einer weiteren Ausführungsform sind die Verbindungselemente zentral und getrennt von dem äußeren zylindrischen Gehäuse der Aufsteckeinheit oder eines Kappenmantel bzw. äußeren zylindrischen Ansatzes eines Ventilelementes vorgesehen. Diese Variante wird nachstehend beschrieben.

In einer Ausführungsform ist das wenigstens eine erste Verbindungselement im Bereich der Wand des rohrförmigen Gehäuses vorgesehen und das wenigstens eine zweite Verbindungselement an korrespondierender Stelle im Bereich eines Randes der Ventileinheit auf der zu der Aufsteckeinheit weisenden Seite der Ventileinheit.

In einer Ausführungsform ist das wenigstens eine erste Verbindungselement im Bereich des Hohlraums des rohrförmigen Gehäuses angeordnet und das wenigstens eine zweite Verbindungselement auf der zu der Aufsteckeinheit weisenden Seite der Ventileinheit von dem Rand der Ventileinheit beabstandet angeordnet oder anders ausgedrückt, korrespondierend zu der Anordnung des ersten Verbindungselementes an der Ventileinheit angeordnet. Bei derartigen Ausführungsformen ist das erste Verbindungselement vorzugsweise über Stege mit der Innenwand des rohrförmigen Gehäuses verbunden.

In einer Ausführungsform ist das wenigstens eine erste Verbindungselement einstückig mit der Aufsteckeinheit ausgeformt und/oder das wenigstens eine zweite Verbindungselement einstückig mit dem Ventildeckel ausgeformt. Eine derartige einstückige Ausgestaltung erhöht die Stabilität der jeweiligen Einheiten des Sprechventils.

In einer Ausführungsform weist entweder die Aufsteckeinheit oder die Ventileinheit mindestens einen Vorsprung und die jeweils andere mindestens eine dazu korrespondierenden Aufnahmeaussparung auf, wobei die Aufsteckeinheit und die Ventileinheit über den Eingriff des Vorsprunges mit der Aufnahmeaussparung beweglich und vorzugsweise lösbar miteinander verbunden sind. Wie bereits erwähnt, können Vorsprung und Aufnahmeaussparung durch ineinander greifende Gewindegänge gebildet werden, es kann sich aber auch um mehrere voneinander beabstandete Rastelemente handeln.

Derart ausgestaltete Verbindungselemente ermöglichen das stufenlose Bewegen bzw. ein abgestuftes Bewegen in definierten Abständen der Dichtfläche an der Ventileinheit von oder zu dem zweiten Ende des Gehäuses der Aufsteckeinheit. Derartige Verbindungen sind auch durch den tracheostomierten Patienten selbst, der eine Tracheostomiekanüle mit einem solchen Sprechventil trägt, leicht bedienbar und erfordern bei der Bedienung keinen Sichtkontakt.

In einer Ausführungsform treten das wenigstens eine erste und wenigstens eine zweite Verbindungselement vollständig voneinander lösbar miteinander in Eingriff. Dadurch wird unabhängig von der Einstellung der ersten und einer zweiten Position das vollständige Abtrennen der Ventileinheit von der Aufsteckeinheit ermöglicht, die so leichter gereinigt werden können.

In einer Ausführungsform besteht die Ventileinheit im Wesentlichen aus einem durchbrochenen Deckel, dessen Innenseite von einer Ventilmembran abgedeckt ist.

Der Ventildeckel weist in einer Ausführungsform außerdem einen zentralen zylindrischen Ansatz auf, der als Verbindungselement mit der Aufsteckeinheit dient. Die Aufsteckeinheit hat auch hier wieder im Wesentlichen die Form eines zylindrischen Rohres, auf dessen eine Stirnseite bzw. auf dessen stirnseitigem Rand der Ventildeckel (in der ersten Position) mit seinem äußeren Rand aufsitzt. Das rohrförmige Aufsteckelement weist zusätzlich ein kleineres, zylindrisches Innenrohr auf, welches über Stege mit der Innenwand des rohrförmigen Gehäuses des Aufsteckelementes verbunden ist. Der zentrale zylindrische Ansatz des Ventildeckels und das über Stege gehaltene Innenrohr des Aufsteckelementes bilden Verbindungselemente, über welcher Ventileinheit und Aufsteckelement miteinander verbunden sind, wobei diese Verbindungselemente wieder mit Rastelementen oder auch mit Innen- bzw. Außengewinden versehen sein können, sodass durch eine Relativdrehung eine axiale Verschiebung und damit ein axiales Abheben des Ventildeckels vom Rand des Aufsteckelementes möglich ist, sodass eine Öffnung zwischen Ventildeckel und Rand des Aufsteckelementes freigegeben wird.

Es versteht sich, dass in kinematischer Umkehr das Ventilelement einen zentralen Rohransatz aufweisen könnte, während im Inneren des rohrförmigen Aufsteckelementes ein über Stege gehaltener Zapfen vorgesehen sein könnte, der in den Rohransatz des Ventildeckels eingreift.

In einer Ausführungsform ist also das erste oder das zweite Verbindungselement als Zylinder und das jeweils andere als Hohlzylinder ausgestaltet, deren Längsachsen auf der Längsachse des rohrförmigen Gehäuses verlaufen und die mit einer Schraub- oder Rastverbindung oder einer Kombination davon miteinander in Eingriff treten, wobei der Zylinder in den Hohlzylinder eingebracht ist oder wird.

Der Begriff "Zylinder", wie er in diesem Zusammenhang hier verwendet wird, bezeichnet einen zylindrischen Körper mit im Wesentlichen kreisförmigen Querschnitt oder einen Hohlzylinder mit im Wesentlichen ringförmigen Querschnitt.

In einer Ausführungsform ist in der Nähe des zweiten Endes in dem Strömungsquerschnitt des rohrförmigen Gehäuses der Aufsteckeinheit ein luftdurchlässiges, Feuchtigkeit und Wärme speicherndes Material , zum Beispiel in Form einer Scheibe angeordnet, das hier auch als "Filterelement" bezeichnet wird. ein solches Material ist vorzugsweise elastisch oder plastisch verformbar und kann zum Beispiel, wenn es die Form einer Kreisscheibe hat, in einer umlaufenden Nut oder einem Falz in der Innenfläche der rohrförmigen Aufsteckeinheit aufgenommen sein ein. Es versteht sich, dass der Durchmesser der Kreisscheibe dann an den Innendurchmesser der entsprechenden Nut oder des Falzes angepasst ist. Eine entsprechende Scheibe hat zum Beispiel 15 bis 25 mm Durchmesser und eine Dicke zwischen 1 und 8 mm, vorzugsweise etwa 3 mm. Das Filterelement kann dabei auch teilweise über das zweite Ende des rohrförmigen Gehäuses in Richtung der Ventileinheit hinausragen oder es schließt mit dem zweiten Ende des rohrförmigen Gehäuses ab oder es ist im Bereich des zweiten Endes des rohrförmigen Gehäuses so angeordnet, dass es in einem Abstand zum zweiten Ende des rohrförmigen Gehäuses angeordnet ist. Der Abstand beträgt vorzugsweise nicht mehr als 3 mm, sollte aber ausreichend bleiben, damit die darüber liegende Membran des Ventils genügend Bewegungsspiel hat, um das Einströmen von Atemluft zuzulassen. Bei dem Filtermaterial handelt sich zum Beispiel um ein schwammartiges Material, das vorzugsweise aus einem offenporigen Kunststoff oder auch aus einem speziellen Papier bestehen kann. In einer Ausführungsform ist das Material hydrophil bzw. hygroskopisch z. B. mit CaCl₂ beschichtet, um die Fähigkeit zur Feuchtigkeitsspeicherung zu verbessern.

Für eine Ausführungsform eines Sprechventils, bei dem das erste und zweite Verbindungselement im Zentrum des rohrförmigen Gehäuses verlaufen, weist das scheibenförmige Filterelement vorzugsweise eine zentrale Durchbrechung auf und ist um das erste und/oder zweite Verbindungselement herum angeordnet.

In einer Ausführungsform weist die Ventileinheit einen scheibenförmigen Ventildeckel und eine Ventilmembran auf, wobei die nach außen weisende Seite und die Umfangsfläche der Ventileinheit durch den Ventildeckel gebildet werden und an einer zu der Aufsteckeinheit weisenden Seite des Ventildeckels eine Ventilmembran an dem Ventildeckel befestigt ist, der einen Ventilsitz bildet und der in einem Bereich, in dem die Ventilmembran anliegt, abschnittsweise durchbrochen ist. Durch eine derartige Anordnung wird gewährleistet, dass dann, wenn die Ventileinheit in der ersten Position zu der Aufsteckeinheit angeordnet ist, Luft durch die Aussparungen/Durchbrechungen in dem Ventildeckel an der Ventilmembran vorbei in das Innere des rohrförmigen Gehäuses und dadurch letztlich in die Tracheostomiekanüle und schließlich in die Lunge des Patienten geführt werden kann. Umgekehrt bewirkt ein Luftstrom in die Gegenrichtung, dass die Ventilmembran gegen den Ventilsitz gedrückt wird und die abschnittsweisen Durchbrechungen verdeckt. Dadurch wird ein Ausströmen von Luft durch das Sprechventil verhindert. Der Ventilsitz ist in einer Ausführungsform als ein die Durchbrechungen im Ventildeckel umgebender, ringförmiger Steg vorgesehen, der den Ventilsitz bildet und der gleichzeitig eine gewisse Vorspannung der Ventilmembran in die geschlossene Position bewirkt, die zum Beispiel als eben Scheibe aus elastischem Material zentral an der Innenfläche des Ventildeckels befestigt ist.

Ein "scheibenförmiger Ventildeckel", wie hier verwendet, bezeichnet ein Element, das im Wesentlichen die Form eines flachen Zylinders aufweist, wobei dessen Dicke bzw. Höhe geringer ist als sein Radius. Es kann sich dabei um einen Kreiszylinder aber auch einen Zylinder mit mehreckiger Grundfläche, wie einer viereckigen, sechseckigen oder achteckigen Grundfläche, handeln. Zudem umfasst dieser Begriff wie er hier verwendet wird auch, dass der scheibenförmige Ventildeckel eine Wölbung aufweisen kann. In einer Ausführungsform weist der Ventildeckel eine konkave Wölbung der zu der Aufsteckeinheit weisenden Seite und gegebenenfalls eine konvexe Wölbung der nach außen weisenden Seite. Der scheibenförmige Ventildeckel kann weiter Verdickungen, Rillen oder andere Vertiefungen oder umlaufende Dickenminderungen aufweisen und kann beispielsweise im Wesentlichen wie ein Frisbee geformt sein.

Vorzugsweise erstreckt sich die Ventilmembran nicht bis zum äußersten Rand der zur der Aufsteckeinheit weisenden Seite des Ventildeckels, wobei in der ersten Position der Ventileinheit der Rand der zu der Aufsteckeinheit weisenden Seite des Ventildeckels an dem zweiten Ende der rohrförmigen Gehäuses luftdicht anliegt.

In einer Ausführungsform weist die Ventilmembran eine Materialstärke von 0,1 mm bis 1 mm, vorzugsweise 0,2 mm bis 0,5 mm auf. Die Membran ist in einer Ausführungsform aus Silikon, EVA, SEBS oder einem anderen thermoplastischen Material hergestellt.

In einer Ausführungsform weist die Ventilmembran Randbereiche und einen zentralen Bereich auf und ist mit dem zentralen Bereich an dem Ventildeckel so angebracht, dass sich die Randbereiche in Richtung der Aufsteckeinheit von dem Ventildeckel abheben können. Durch eine derartige Ausgestaltung ist die oben beschriebene Ventilwirkung gewährleistet. In einer Ausführungsform liegt dabei die Ventilmembran mit ihrem Randbereich mit Vorspannung an der zu der Aufsteckeinheit weisenden Seite des Ventildeckels an. Eine derartige Vorspannung gewährleistet, dass die Ventilmembran an dem Bereich des Ventildeckels, der Durchbrechungen aufweist, eng anliegt., wobei zum Beispiel eine Druckdifferenz von etwa 5 mbar zum Öffnen des Ventils benötigt wird

In einer Ausführungsform stellt das zweite Verbindungselement, das mit dem Ventildeckel verbunden ist, gleichzeitig eine Befestigung der Membran an dem Ventildeckel bereit.

In einer Ausführungsform sind die Verbindungselemente der Ventileinheit und der Aufsteckeinheit auf der Längsachse des Gehäuses angeordnet und die Membran in einem um die Längsachse verlaufenden Bereich an dem Ventildeckel befestigt, wobei sowohl die Befestigung der Membran als auch die Verbindungselemente als Hohlzylinder ausgestaltet sind und der Ventildeckel eine zentrale Aussparung aufweist, wobei eine hohlzylinderförmige Struktur mit offenen Enden gebildet wird, die durch den Ventildeckel und die gesamte Ventileinheit verläuft und in der Aufsteckeinheit endet. Durch eine derartige Ausgestaltung wird innerhalb des Ventildeckels eine Öffnung für den Anschluss eines Sauerstoffschlauches bereitgestellt, die bei normalem Gebrauch des Sprechventils durch ein Verschlusselement, wie beispielsweise einen Stopfen, verschlossen ist. In einer Ausführungsform besteht der Ventildeckel aus einem elastischen Material und liegt in der ersten Position der Ventileinheit mit einer Vorspannung an dem zweiten Ende des Gehäuses luftdicht an, wobei der Ventildeckel bei einem Überdruck in dem Sprechventil partiell nach außen gedrückt wird, so dass dessen Rand nicht mehr an dem zweiten Ende des rohrförmigen Gehäuses anliegt. Die Vorspannung kann beispielsweise so gewählt werden, dass eine Druckdifferenz von etwa 30 bis 50 mbar erforderlich ist, um den Deckel (Ventilsitz) von seinem Eingriff mit dem Ende des rohrförmigen Gehäuses der Aufsteckeinheit abzuheben. Bei einer derartigen Ausgestaltung kann der Ventildeckel als eine Art "Überdruckventil" fungieren, das bei starkem Innendruck in dem Sprechventil entgegen der elastischen Vorspannungen nach außen wegklappt und dadurch ein Entweichen von Luft ermöglicht.

Der Begriff "in dem Sprechventil" bezeichnet das Lumen des Gehäuses, das in der ersten Position der Ventileinheit an dem zweiten Ende durch die Ventileinheit begrenzt ist und an dem ersten Ende in das Lumen der Tracheostomiekanüle übergeht.

In einer Ausführungsform ist die Umfangsfläche der Ventileinheit mit einer Riffelung, Aufrauung und/oder einem Vorsprung oder Vertiefung in der Umfangsfläche ausgestattet. Derartige Aufrauungen bzw. Riffelungen oder Vorsprünge und Vertiefungen erleichtern das Ergreifen der Ventileinheit bzw. des Ventildeckels an der Umfangsfläche und damit die Handhabung des Sprechventils beim Bewegen der Ventileinheit von der ersten in die wenigstens eine zweite Position oder umgekehrt. Zudem kann ein an einer einzigen Stelle in der Umfangsfläche angebrachte Vertiefung oder ein Vorsprung insbesondere bei einer Schraubverbindung zwischen Ventildeckel und Gehäuse der Aufsteckeinheit einen ertastbaren Indikator für den Öffnungszustand des Sprechventils bereitstellen. Ein an einer Stelle angebrachter Vorsprung kann in bestimmten Ausführungsformen die Form einer Öse, Lasche, Halbkugel, o. ä. haben.

In einer Ausführungsform weist die Ventileinheit Rastelemente auf, die vorzugsweise hörbar einrasten. Zusätzliche können eine oder mehrere von außen sichtbare oder fühlbare Markierungen vorgesehen sein, anhand derer man die Position des Sprechventils erkennen kann.

In einer Ausführungsform weisen das Gehäuse der Aufsteckeinheit und die Umfangsfläche der Ventileinheit zusammen eine sich konisch nach außen verjüngende Form auf. Eine derartige Form kann bei entsprechender Anpassung an gängige Maße von weiblichen Anschlüssen von Beatmungsschläuchen einen Sauerstoffanschluss bereitstellen, mit Hilfe dessen der Patient mit Sauerstoff versorgt werden kann. Bei aufgestecktem Sauerstoffanschluss ist ein Entweichen des Sauerstoffs durch die oberen Atemwege vorgesehen. In einer Ausführungsform beträgt das Kegelverhältnis der sich nach außen konisch verjüngenden Form 1:40, wobei ein äußerer Durchmesser des Gehäuses der Aufsteckeinheit an einer Stelle 22 mm beträgt. Vorzugsweise befindet sich die Stelle im Wesentlichen in der Mitte zwischen dem ersten und dem zweiten Ende, wobei Abweichungen von der Mitte von bis zu +/- 3 mm möglich sind.

In einer Ausführungsform ist das erste Ende der Aufsteckeinheit reibschlüssig auf den Konnektor an der Tracheostomiekanüle aufsteckbar. Tracheostomiekanülen sind für das Aufstecken von Sauerstoffanschlüssen oder weiterem Zubehör mit einem Konnektor versehen, der Maße aufweist, die ein derartiges Aufstecken ermöglichen. Vorzugsweise ist die Innenwand des Gehäuses der Aufsteckeinheit an derartige Bemaßungen angepasst.

Die eingangs genannte Aufgabe wird ebenfalls gelöst durch eine Tracheostomiekanüle mit einem Sprechventil entsprechend den oben beschriebenen Varianten.

Weitere Vorteile, Merkmale und Anwendungsmöglichkeiten der vorliegenden Erfindung werden deutlich anhand der folgenden Beschreibung bevorzugter Ausführungsformen und den dazugehörigen Figuren. Es zeigen:
- Figur 1:: eine erste Ausführungsform eines erfindungsgemäßen Sprechventils in der ersten Position der Ventileinheit in verschiedenen Ansichten,
- Figur 2:: die in Figur 1 gezeigte Ausführungsform in einer zweiten Position der Ventileinheit in verschiedenen Ansichten,
- Figur 3:: eine weitere Ausführungsform eines erfindungsgemäßen Sprechventils ohne und mit Tracheostomiekanüle in verschiedenen Ansichten ,
- Figur 4:: eine weitere Ausführungsform eines erfindungsgemäßen Sprechventils, die auf eine Tracheostomiekanüle aufgesteckt ist,
- Figur 5:: eine perspektivische Ansicht einer weiteren Ausführungsform teilweise im Schnitt,,
- Figur 6:: einen die Achse enthaltenden Schnitt der Ausführungsform nach Figur 5,
- Figur 7:: eine Seitenansicht des Sprechventils nach Figur 5 in einem teilweise geöffneten Zustand und
- Figur 8:: eine Seitenansicht des Sprechventils nach Figur 5 in einem weitgehend geschlossenem Zustand.

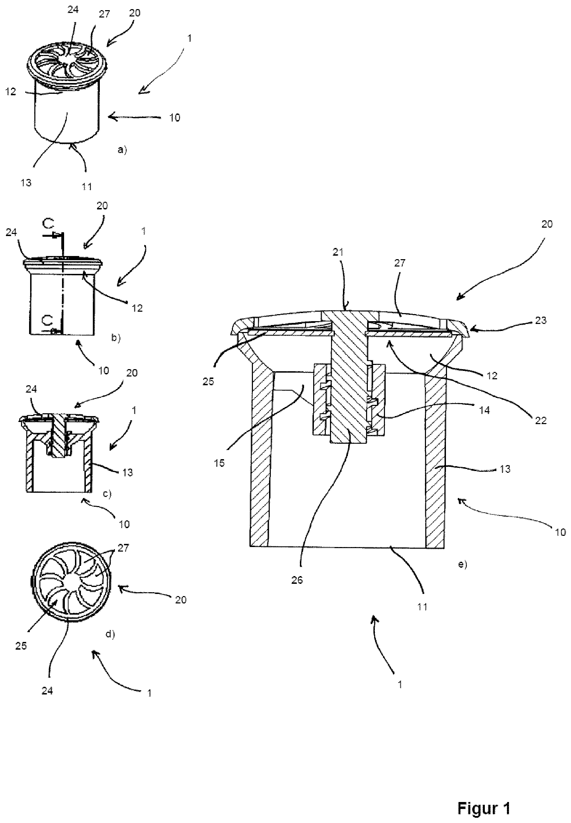

In Figur 1 ist ein erfindungsgemäßes Sprechventil in der ersten Position der Ventileinheit dargestellt. Dabei ist Figur 1a eine perspektivische Darstellung eines solchen Sprechventils, Figur 1b eine seitliche Draufsicht auf das Sprechventil, Figur 1c eine Schnittdarstellung entlang der Linie C-C, Figur 1d eine Draufsicht auf die nach außen weisende Seite des Ventildeckels und Figur 1e eine vergrößerte Darstellung von Figur 1c.

Bei dieser Ausführungsform des Sprechventils 1 weist die Ventileinheit 20 einen Ventildeckel 24 und eine Ventilmembran 25 auf, wobei die Ventilmembran in einem mittleren Bereich mit dem Ventildeckel verbunden ist und dessen Rand der zu der Aufsteckeinheit 10 weisenden Seite 22 nicht bedeckt. Der Ventildeckel 24 der Ventileinheit 20 liegt in der ersten Position dicht an dem zweiten Ende 12 des Gehäuses 13 an. Das zweite Ende 12 des Gehäuses 13 ist bei dieser Ausführungsform mit einem sich konisch weitender Endabschnitt ausgestaltet, an den der Ventildeckel 24 mit dem Rand seiner zu der Aufsteckeinheit 10 weisenden Seite anliegt 22, wobei der Rand des Ventildeckels 24 abgestuft ist und sich im Bereich der Anlagefläche an das zweite Ende des rohrförmigen Gehäuses 13 der Aufsteckeinheit 10 verjüngt.

Das rohrförmige Gehäuse 13 und der Ventildeckel 24 weisen je ein Verbindungselement 14 bzw. 26 auf, die im Wesentlichen zylinderförmig sind und auf der Längsachse des rohrförmigen Gehäuses angeordnet sind. Das erste Verbindungselement 14 des rohrförmigen Gehäuses ist mit Stegen 15 an der Innenwand des rohrförmigen Gehäuses 13 befestigt. Insgesamt stellen das erste Verbindungselement und zweite Verbindungselement eine Schraubverbindung bereit. Das zweite Verbindungselement 26 an dem Ventildeckel fixiert gleichzeitig die Ventilmembran 25 an der zu der Aufsteckeinheit weisenden Seite des Ventildeckels 24. Der Ventildeckel weist Durchbrechungen 27 auf, die wenn innerhalb des röhrenförmigen Gehäuses des Aufsteckabschnitts kein Unterdruck im Vergleich zu außen vorliegt, durch die an dem Ventildeckel (mit Vorspannung?) anliegende Membran 25 abgedeckt werden.

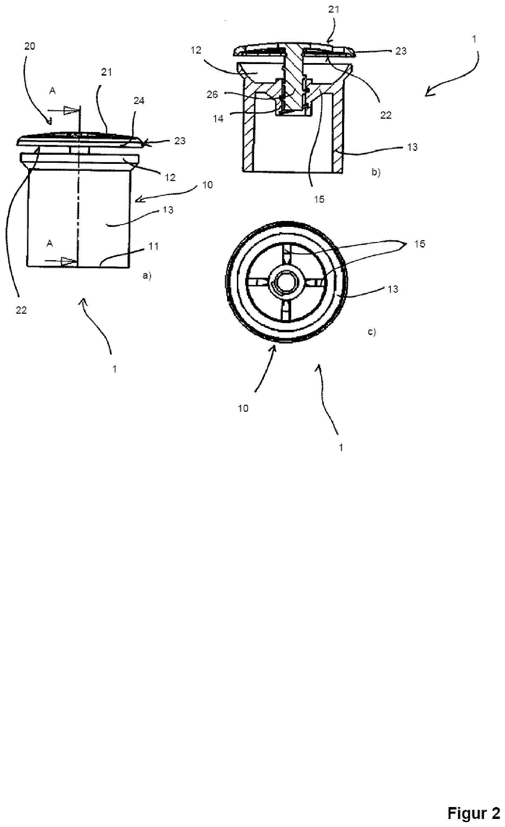

Figur 2 zeigt das in Figur 1 dargestellte Sprechventil 1 in einer zweiten Position der Ventileinheit 20. Figur 2a ist eine seitliche Draufsicht auf das Sprechventil, Figur 2b eine Schnittdarstellung entlang der Linie A-A und Figur 2c eine Draufsicht von außen auf das zweite Ende 12 des rohrförmigen Gehäuses 13, wobei die Ventileinheit vollständig entfernt ist.

In einer zweiten Position der Ventileinheit 20 ist der Ventildeckel 24 von dem zweiten Ende 12 der rohrförmigen Gehäuses 13 beabstandet, so dass ein Zwischenraum zwischen Ventileinheit und Aufsteckeinheit entsteht. In dieser Position ist ein Gasaustausch durch den Zwischenraum zwischen Ventileinheit 20 bzw. Ventildeckel 24 und Aufsteckeinheit 10 möglich, wobei sowohl Luft in die Aufsteckeinheit als auch aus dieser heraus gelangen kann und dadurch letztlich eine Atmung ermöglicht wird, wie es auch bei einer Tracheostomiekanüle ohne aufgebrachtes Sprechventil der Fall ist. In dieser Position der Ventileinheit 20 wird die Wirkung der Ventileinheit 20 umgangen.

In Figur 2b ist deutlich zu erkennen, dass zum Einstellen der gezeigten zweiten Position die Ventileinheit 20 mit ihrem zweiten Verbindungselement 26 um eine halbe Windung aus dem ersten Verbindungselement 14 herausgeschraubt wurde. Ein weiteres Herausschrauben aus dem ersten Verbindungselement 14 stellt weitere zweite Positionen bereit, in denen der Abstand zwischen Ventileinheit 20 und Aufsteckeinheit 10 größer ist als in der gezeigten Figur 2. Letztlich ist auch ein vollständiges Herausschrauben möglich, wodurch die in Figur 2c gezeigte Draufsicht auf das zweite Ende 12 sichtbar wird.

In Figur 2c ist deutlich zu erkennen, dass das erste Verbindungselement 14 auf der Längsachse des röhrenförmigen Gehäuses 13 verläuft und wie das röhrenförmige Gehäuse selbst eine Rotationssymmetrie aufweist. Das Verbindungselement ist mit vier Stegen 15 an der Innenwand des rohrförmigen Gehäuses 13 befestigt. Gehäuse 13, Stege 15 und das erste Verbindungselement 14 bilden zusammen die Aufsteckeinheit 10.

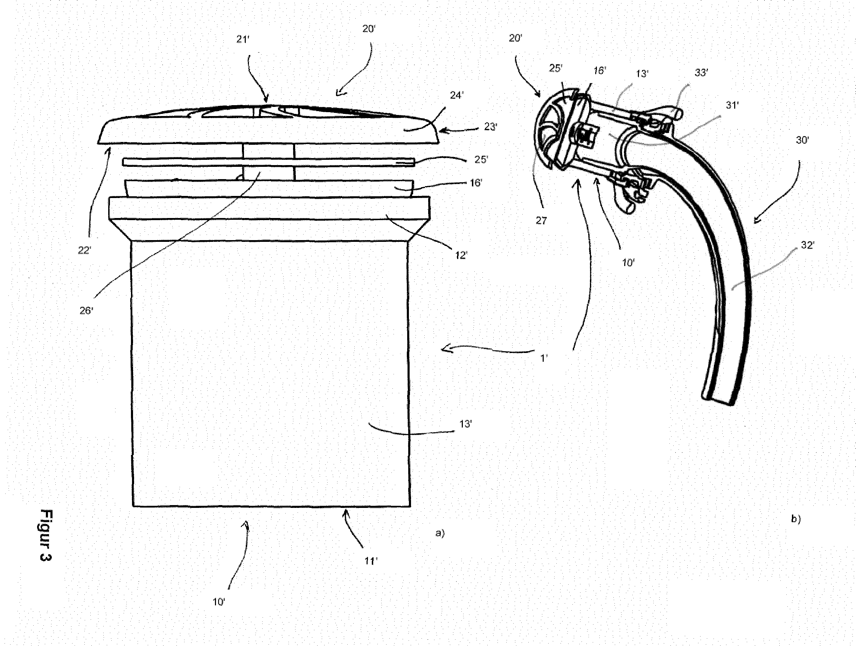

In Figur 3 ist eine weitere Ausführungsform eines erfindungsgemäßes Sprechventils 1' gezeigt, wobei Figur 3a eine seitliche Draufsicht auf das Sprechventil in einer starken Vergrößerung zeigt und Figur 3b eine perspektivische Ansicht einer längs aufgeschnittenen Tracheostomiekanüle mit einem Sprechventil entsprechend der Ausführungsform in Figur 3a. In beiden Darstellungen befindet sich die Ventileinheit 20' in einer zweiten Position zu der Aufsteckeinheit 10'. Dieses Sprechventil 1' weist neben den bereits beschriebenen Bestandteilen zusätzlich ein Filterelement 16' auf, das innerhalb des röhrenförmigen Gehäuses 13' im Bereich des zweiten Endes 12' angeordnet ist und über dieses hinausragt.

Bei dieser Ausführungsform ist das Filterelement 16' an dem zweiten Verbindungselement 26 befestigt und bewegt sich mit diesem wenn eine Bewegung von der ersten in eine zweite Position erfolgt. Dasselbe gilt für die Ventilmembran, die in einem Abstand zu dem Filterelement an dem zweiten Verbindungselement angebracht ist und um das zweite Verbindungselement an dem Ventildeckel verläuft. Das Filterelement ist ein scheibenförmiges Material mit zentraler Durchbrechung, durch die das zweite Verbindungselement geführt ist. Ein Austausch des Filterelements ist durch vollständiges Entfernen der Ventileinheit von der Aufsteckeinheit möglich. Das Filterelement besteht aus einem porösen Kunststoff und nimmt, wenn die Ventileinheit in einer zweiten Position zu der Aufsteckeinheit angeordnet ist, Feuchtigkeit aus der durch das Sprechventil ausgeatmeten Luft auf. Diese wird bei anschließendem Einatmen sukzessive wieder an die Einatemluft abgegeben, wodurch eine Befeuchtung der Einatemluft erfolgt.

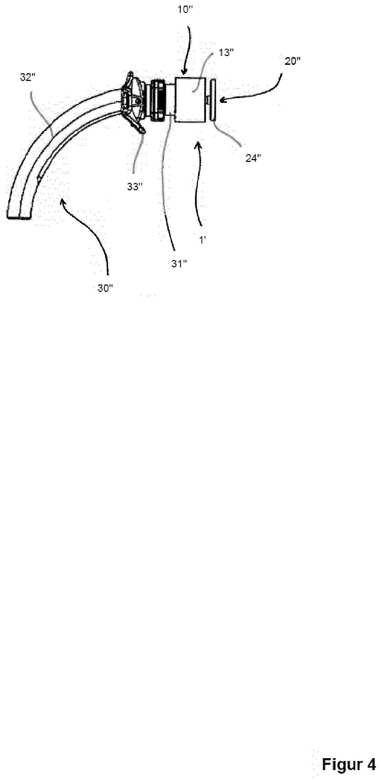

In Figur 4 ist eine weitere Ausführungsform eines erfindungsgemäßen Sprechventils 1" in einer seitlichen Draufsicht gezeigt, das auf eine Tracheostomiekanüle aufgesteckt ist. Die Ventileinheit 20" befindet sich dabei in einer zweiten Position zu der Aufsteckeinheit 10". Bei dieser Ausführungsform ist die Aufsteckeinheit 10" so bemessen, dass sie von ihrem ersten zu ihrem zweiten Ende innen und außen konisch zuläuft und die Innenwand das weibliche Element eines 15 mm Konnektors bildet, wodurch ein reibschlüssiges Aufstecken auf einen üblicherweise an einer Tracheostomiekanüle angebrachten 15 mm Konnektor 31" möglich ist. Die Tracheostomiekanüle 30" verfügt über ein Kanülenrohr 32" mit Innen- und Außenkanüle, wobei die Innen- und Außenkanüle durch eine Überwurfmutter miteinander verbunden sind. Weiter befindet sich an der Tracheostomiekanüle ein Kanülenschild 33", mit Hilfe dessen eine Befestigung an dem Hals eines Patienten ermöglicht wird und gleichzeitig Druckstellen durch die Tracheostomiekanüle vermieden werden. Das in dieser Darstellung gezeigte Sprechventil 1" ist weiter so gestaltet, dass das röhrenförmige Gehäuse des Aufsteckelements 10" auch äußerlich einen Konus bildet und zusammen mit der Ventileinheit eine konische Form darstellt, die einem männlichen Element eines 22 mm Konnektors entspricht. Durch diese Ausgestaltung wird ein Aufstecken eines mit einem weiblichen 22 mm Konnektor versehenen Beatmungsschlauchs möglich.

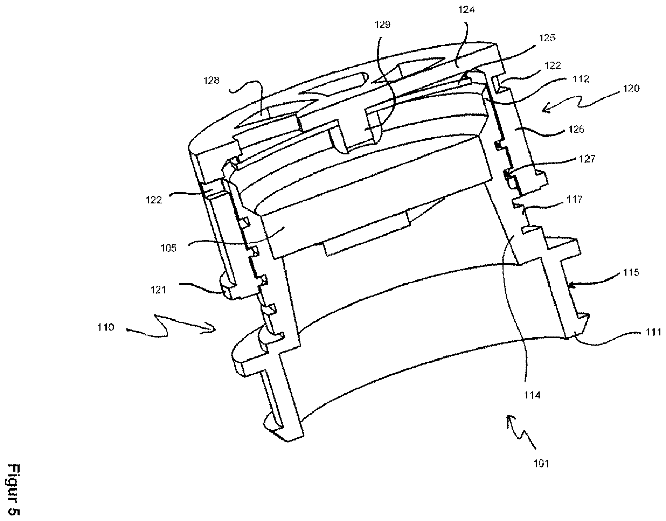

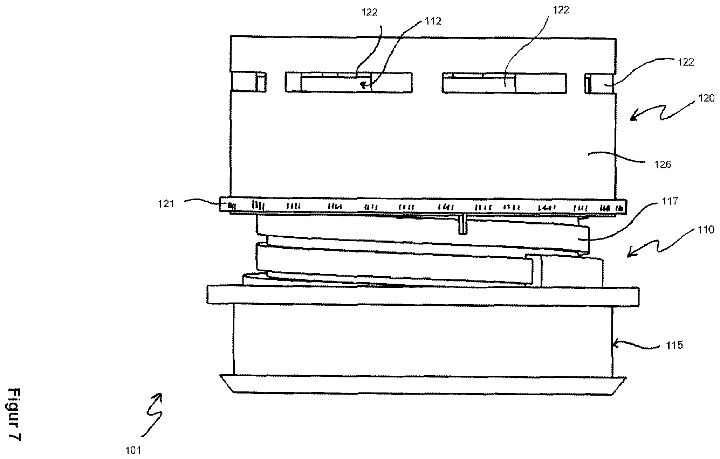

Das Sprechventil 101 gemäß den Figuren 5 bis 8 besteht aus einer Aufsteckeinheit 110, die als ein mehr oder weniger zylindrisches Gehäuse 114 ausgebildet ist, welches ein erstes Ende 111 und ein zweites Ende 112 aufweist. Ein mittlerer Abschnitt des Gehäuses 114 ist als Verbindungselemente ausgebildet, in dem die zylindrische Außenseite des Gehäuses 114 in diesem Bereich mit einem Außengewinde 117 versehen ist. Die Ventileinheit 120 hat die Form einer Kappe mit einem Ventildeckel 124 und einem Kappenmantel 126. Der Kappenmantel 126 dient gleichzeitig als Verbindungselement, in dem auf seiner Innenfläche ein Innengewinde 127 vorgesehen ist, welches passend in das Außengewinde des Gehäuses 114 eingreift. Der Ventildeckel 124 der Ventileinheit weist Durchbrechungen 128 auf. Außerdem erstreckt sich von dem Ventildeckel 124 einwärts ein Haltezapfen 129, der zentral an der Innenseite des Ventildeckels 124 angeordnet ist und der beispielsweise mit Hilfe einer Gewindehülse 121 eine elastisch bewegbare Ventilmembran an der Unterseite des Ventildeckels festhält.

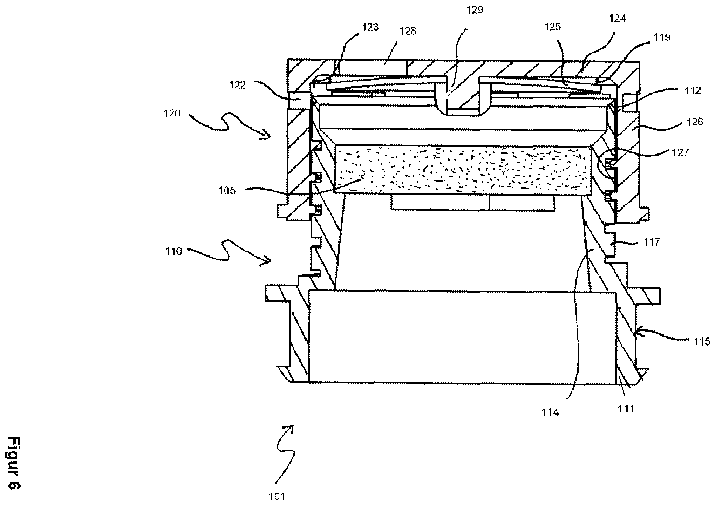

Wie man insbesondere in Figur 6 erkennen kann, weist die kappenförmige Ventileinheit 120 auf ihrer Innenseite am Übergang von dem Kappenmantel 120 zum Ventildeckel 124 eine konische Übergangsfläche auf, die als Dichtfläche 123 dient, wobei das zweite Ende 112 des Aufsteckteils 110 als Dichtlippe 112' ausgebildet ist, in dem die obere innere Kante des rohrförmigen Gehäuses 116 abgeschrägt ist. Wenn die Ventileinheit 120 vollständig auf die Aufsteckeinheit 110 aufgeschraubt worden ist, steht die Dichtlippe 112' mit der konischen Dichtfläche 123 in Dichteingriff und blockiert damit jeglichen Durchgang von der Außenseite über die Aussparungen 122 in das innere Lumen der Aufsteckeinheit 110.

In den Figuren 5 ,6 und 7 ist eine Position der Ventileinheit 120 relativ zur Aufsteckeinheit 110 dargestellt, in welcher die Dichtlippe 112' die Aussparungen 122 teilweise freigibt, sodass Luft in beide Richtungen, d.h. sowohl zum Einatmen als auch zum Ausatmen, durch die Aussparungen 122 und das Lumen der Aufsteckeinheit 110 hindurchströmen kann. Jegliche Luft, die durch die Aufsteckeinheit 110 hindurchtritt, muss ein Filterelement 105 passieren, welches in den Formen einer Scheibe, die beispielsweise zwischen 3 und 5 mm dick sein kann, den gesamten Querschnitt des Lumens der Aufsteckeinheit 110 abdeckt. Das Filter- bzw. Befeuchtungselement 105 besteht beispielsweise aus einem porösen Schwamm- oder Kunststoffmaterial und kann wahlweise auch ein mehrlagiges Papiermaterial sein, welches eine hydrophile bzw. hygroskopische Beschichtung aufweist. Der Strömungswiderstand durch das Filterelement 105 sollte möglichst gering sein, gleichzeitig sollte das Filterelement jedoch beim Ausatmen durch die Aussparungen 122 des Sprechventils Feuchtigkeit und Wärme der Atemluft aufnehmen und diese Feuchtigkeit und Wärme zumindest teilweise an die eingeatmete Luft abgeben. Im Gegensatz zu der vorher beschriebenen Ausführungsform gemäß den Figuren 1 und 2 benötigt das Filterelement hier keine Durchbrechung und sitzt, vorzugsweise unter elastischer radialer Spannung in einem Falz an der Innenseite de Gehäuses 114.

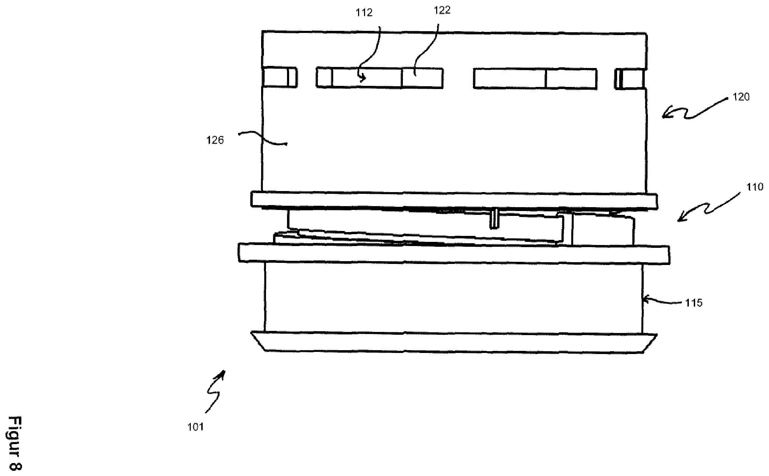

Im geschlossenen Zustand, wie er beispielsweise in Figur 8 wiedergegeben ist und in welchem die Öffnungen 122 durch die Dichtlippe 112 verschlossen sind, funktioniert das Sprechventil in der Weise, dass beim Einatmen aufgrund der Druckdifferenz zwischen der Umgebung und dem Lumen der Aufsteckeinheit 110 die Ventilmembran 125 von dem Dichtungssteg 119 abhebt und dadurch die Atemluft durch die Aussparungen 128 über die Ventilmembran 125 hinweg und zwischen dem sich zwischen dem Dichtungssteg 119 und der Ventilmembran 125 bildenden Spalt außen an der Ventilmembran 125 vorbei in das Lumen der Aufsteckeinheit 110 einströmen kann.

Beim Ausatmen wird durch den Überdruck innerhalb des Lumens 110 die Ventilmembran 125 an den umlaufenden Dichtungssteg 119 angedrückt, sodass der Zugang zu den Aussparungen 128 blockiert ist und die Atemluft nicht über das Ventil entweichen kann, sondern über die natürlichen Atemwege nach außen gelangen muss, sodass der Patient dadurch die Möglichkeit hat, zu sprechen. Im Übrigen ist es aber auch möglich, durch ein teilweises Öffnen des Ventils, beispielsweise in die in den Figuren 5 bis 7 dargestellte Position, den Öffnungsspalt zu den Aussparungen 122 so einzustellen, dass jeweils ein Teil der Atemluft durch die Aussparungen 122 ein- und ausströmt, jedoch ein weiterer Teil der Luft, jedenfalls beim Ausatmen, auch über die natürlichen Atemwege strömt.

Die Innenwand des Gehäuses 114 weist im oberen Bereich einen Falz auf, der eine Aussparung für die Aufnahme des Randes des scheibenförmigen Filterelementes 105 bildet, sodass das Filterelement 105 in dem Gehäuse 114 nicht verrutschen kann.

Die Ventilmembran 125 ist an einem zentralen Zapfen 129 befestigt, der sich vom Zentrum des Ventildeckels 124 abwärts erstreckt.

Für Zwecke der ursprünglichen Offenbarung wird darauf hingewiesen, dass sämtliche Merkmale, wie sie sich aus der vorliegenden Beschreibung, den Zeichnungen und den abhängigen Ansprüchen für einen Fachmann erschließen, auch wenn sie konkret nur im Zusammenhang mit bestimmten weiteren Merkmalen beschrieben wurde, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen und die Betonung der Unabhängigkeit der einzelnen Merkmale voneinander wird hier nur der Kürze und der Lesbarkeit der Beschreibung wegen verzichtet.

Die Erfindung ist nicht auf die offenbarten Ausführungsformen beschränkt. Abwandlungen der offenbarten Ausführungsformen sind für den Fachmann aus den Zeichnungen, der Beschreibung und den beigefügten Ansprüchen offensichtlich.

### Bezugszahlen:

- 1, 1', 1": Sprechventil
- 10, 10', 10": Aufsteckeinheit
- 11, 11', 11": erstes Ende
- 12, 12', 12": zweites Ende
- 13, 13', 13": Gehäuse
- 14, 14', 14": erstes Verbindungselement
- 15, 15', 15": Stege
- 16, 16', 16": Filterelement
- 20, 20', 20": Ventileinheit
- 21, 21', 21": nach außen weisende Seite
- 22, 22', 22": zur Aufsteckeinheit weisende Seite
- 23, 23', 23": Umfangsfläche
- 24, 24', 24": Ventildeckel
- 25, 25', 25": Ventilmembran
- 26, 26', 26": zweite Verbindungselement
- 27, 27', 27": Durchbrechungen
- 30, 30', 30": Tracheostomiekanüle
- 31, 31', 31 ": Konnektor
- 32, 32', 32": Kanülenrohr
- 33, 33', 33": Kanülenschild

## Patentansprüche

1. Sprechventil (1, 1', 1", 101) zum Aufsetzen auf eine Tracheostomiekanüle,
das eine Ventileinheit (20, 20', 20", 120) und eine Aufsteckeinheit (10, 10', 10", 110) umfasst,
wobei die Aufsteckeinheit ein rohrförmiges Gehäuse (13, 13', 13", 114) aufweist, mit einem ersten (11, 11', 111) und einem zweiten Ende (12, 12', 112) und einer vom ersten (11, 11', 111) zum zweiten Ende (12, 12', 112) verlaufenden Längsachse,
wobei das erste Ende (11, 11', 111) der Aufsteckeinheit (10, 10', 10", 110) mit einem Konnektor an der Tracheostomiekanüle verbindbar ist und die Ventileinheit (20, 20', 20", 120) an dem zweiten Ende (12, 12', 112) der Aufsteckeinheit (10, 10', 10", 110) angeordnet ist und wobei die Ventileinheit (20, 20', 20", 120) und die Aufsteckeinheit (10, 10', 10", 110) beweglich und vorzugsweise lösbar miteinander verbunden sind,
**dadurch gekennzeichnet, dass**
die Ventileinheit so ausgestaltet und angeordnet ist, dass sie
in einer ersten Position das Gehäuse (13, 13', 13", 114) der Aufsteckeinheit (10, 10', 10", 110) bezüglich einer Strömungsrichtung luftdicht verschließt und
in wenigstens einer zweiten Position einen Luftstrom in das Gehäuse (13, 13', 13", 114) und aus diesem heraus zulässt,
wobei die Ventileinheit (20, 20', 20", 120) durch eine Bewegung parallel zu der Richtung der Längsachse des rohrförmigen Gehäuses (13, 13', 13", 114) von der ersten in die wenigstens eine zweite Position überführbar ist, wobei die Ventileinheit (20, 20', 20", 120) einen scheibenförmigen, durchbrochenen Ventildeckel (24, 24', 24", 124) und eine auf der Innenseite des Ventildeckels (24, 24', 24", 124) dichtend anlegbare elastische Ventilmembran (25, 25', 125) aufweist, welche die Durchbrechungen (27, 27', 128) abdeckt und mindestens bei Überschreiten einer vorgebbaren Druckdifferenz zwischen Außen- und Innenseite der Ventileinheit (20, 20', 20", 120) von einem Ventilsitz an der Innenseite des Ventildeckels (24, 24', 24", 124) abhebt.

2. Sprechventil (1, 1', 1", 101) gemäß Anspruch 1, **dadurch gekennzeichnet, dass** die Ventileinheit (20, 20', 20", 120) mindestens eine Aussparung aufweist, die in der ersten Position durch eine an der Umgebung der Aussparung anliegende Wand der Aufsteckeinheit (10, 10', 10", 110) verdeckt und in der zweiten Position durch die Wand der Aufsteckeinheit freigegeben wird.

3. Sprechventil (1, 1', 1", 101) gemäß Anspruch 1 oder 2, **dadurch gekennzeichnet, dass** die Aufsteckeinheit (10, 10', 10", 110) wenigstens ein erstes Verbindungselement (14, 14') aufweist und die Ventileinheit (20, 20', 20", 120) wenigstens ein zweites Verbindungselement (26, 26') aufweist, wobei entweder das erste (14, 14') oder das zweite Verbindungselement (26, 26') einen Vorsprung und das jeweils andere Verbindungselement eine dazu korrespondierende Aufnahmeaussparung aufweist, wobei die Aufsteckeinheit (10, 10', 10", 110) und die Ventileinheit über die miteinander in Eingriff stehenden ersten (14, 14') und zweiten Verbindungselemente (26, 26') beweglich miteinander verbunden sind.

4. Sprechventil (1, 1', 1", 101) gemäß Anspruch 3, **dadurch gekennzeichnet, dass** derwenigstens eine Vorsprung und die wenigstens eine Aufnahmeausparung jeweils durch ein Schraubgewinde oder durch eine Rastverbindung mit wenigstens zwei Raststufen oder eine Kombination von Gewinde und Rastverbindung gebildet werden.

5. Sprechventil (1, 1', 1", 101) gemäß einem der Ansprüche 2 bis 4, **dadurch gekennzeichnet, dass** die Verbindungselemente einen Innenzylinder und einen hohlen Außenzylinder aufweisen die konzentrisch und passend ineinandergreifen und von denen einer mit der Ventileinheit und der andere mit der Aufsteckeinheit (10, 10', 10", 110) jeweils fest verbunden ist.

6. Sprechventil (1, 1', 1", 101) gemäß Anspruch 5, **dadurch gekennzeichnet, dass** der Zylinder und der Hohlzylinder jeweils einen kleineren Durchmesser aufweisen als das Lumen der rohrförmigen Aufsteckeinheit (10, 10', 10", 110) und jeweils zentral bezüglich der Aufsteckeinheit und der Ventileinheit angeordnet sind

7. Sprechventil (1, 1', 1", 101) gemäß Anspruch 5, **dadurch gekennzeichnet, dass** der Zylinder seinerseits ein Hohlzylinder ist und dass Zylinder und Hohlzylinder durch das rohrförmige Gehäuse (13, 13', 13", 114) der Aufsteckeinheit (10, 10', 10", 110) und einen zylindrischen Mantel einer kappenförmigen Ventileinheit (20, 20', 20", 120) gebildet werden.

8. Sprechventil (1, 1', 1", 101) gemäß einem der vorangehenden Ansprüche, **dadurch gekennzeichnet, dass** im Strömungsquerschnitt des zweiten Endes (12, 12', 112) des rohrförmigen Gehäuses (13, 13', 13", 114) der Aufsteckeinheit ein luftdurchlässiges, Filterelement (16, 16', 105) angeordnet ist, vorzugsweise ein Filterelement (16, 16', 105), das ein Wärme und/oder Feuchtigkeit speicherndes, insbesondere ein hygroskopisches Material aufweist.

9. Sprechventil (1, 1', 1", 101) gemäß einem der vorangehenden Ansprüche, **dadurch gekennzeichnet**, dassdie Ventilmembran (25, 25', 125) Randbereiche und einen zentralen Bereich aufweist, wobei der zentrale Bereich an dem Ventildeckel (24, 24', 24", 124) so befestigt ist, dass sich die Randbereiche der Membran in Richtung der Aufsteckeinheit von dem Ventilsitz bei Einwirken einer entsprechenden Druckkraft elastisch abheben können.

10. Sprechventil (1, 1', 1", 101) gemäß Anspruch 9, **dadurch gekennzeichnet, dass** der Ventildeckel (24, 24', 24", 124) ebenfalls aus einem elastischen Material gebildet ist, und in der ersten Position mit einer Vorspannung an dem zweiten Ende (12, 12', 112) des Gehäuses (13, 13', 13", 114) luftdicht anliegt und in der zweiten Position einen Durchtrittsspalt zu dem oberen Rand des zweiten Endes (12, 12', 112) des rohrförmigen Gehäuses (13, 13', 13", 114) der Aufsteckeinheit freigibt.

11. Sprechventil (1, 1', 1", 101) gemäß einem der vorstehenden Ansprüche, **dadurch gekennzeichnet, dass** die Ventileinheit (20, 20', 20", 120) als Ventilkappe mit einem zylindrischen Kappenmantel und einem scheibenförmigen Ventildeckel (24, 24', 24", 124)ausgebildet ist, wobei vorzugsweise der Kappenmantel mindestens das zweite Ende (12, 12', 112) des rohrförmigen Gehäuses (13, 13', 13", 114) von außen umfasst, wobei eine Dichtfläche auf der Innenseite des Ventildeckels (24, 24', 24", 124) oder am Übergang zwischen Kappenmantel und Ventildeckel (24, 24', 24", 124) vorgesehen ist, die in der ersten Position in dichtenden Eingriff mit dem oberen Rand des zweiten Endes (12, 12', 112) des rohrförmigen Gehäuses (13, 13', 13", 114) steht.

12. Sprechventil (1, 1', 1", 101) gemäß einem der vorstehenden Ansprüche, **dadurch gekennzeichnet, dass** der obere Rand des zweiten Endes (12, 12', 112) des rohrförmigen Gehäuses (13, 13', 13", 114) als Dichtlippe ausgebildet ist.

13. Sprechventil (1, 1', 1", 101) gemäß einem der vorstehenden Ansprüche, **dadurch gekennzeichnet, dass** die Umfangsfläche (23, 23') der Ventileinheit (20, 20', 20", 120) eine Riffelung, Aufrauung und/oder einen Vorsprung oder eine Vertiefung in der Umfangsfläche (23, 23') aufweist und/oder die Ventileinheit (20, 20', 20", 120) auf ihrer nach außen weisenden Seite ein oder mehrere Markierungen aufweist.

14. Sprechventil (1, 1', 1", 101) gemäß einem der vorangehenden Ansprüche, **dadurch gekennzeichnet, dass** zwischen Ventileinheit (20, 20', 20", 120) und Aufsteckeinheit (10, 10', 10", 110) für mindestens eine der ersten und zweiten Positionen Rastelemente vorgesehen sind, die hörbar miteinander verrasten.

15. Sprechventil (1, 1', 1", 101) gemäß einem der vorangehenden Ansprüche, wobei das Gehäuse der Aufsteckeinheit (10, 10', 10", 110) und die Umfangsfläche (23, 23') der Ventileinheit (20, 20', 20", 120) zusammen eine sich konisch nach außen verjüngende Form haben und/oder das erste Ende (11, 11', 111) der Aufsteckeinheit (10, 10', 10", 110) reibschlüssig auf den Konnektor aufsteckbar ist.

16. Tracheostomiekanüle (30', 30") mit einem Sprechventil (1, 1', 1", 101) gemäß einem der vorangehenden Ansprüche.

## Claims

1. A speaking valve (1, 1', 1", 101) for fitment on to a tracheostomy cannula,
which includes a valve unit (20, 20', 20", 120) and an attachment unit (10, 10', 10", 110),
wherein the attachment unit has a tubular housing (13, 13', 13", 114) having a first end (11, 11', 111) and a second end (12, 12', 112) and a longitudinal axis extending from the first end (11, 11', 111) to the second end (12, 12', 112),
wherein the first end (11, 11', 111) of the attachment unit (10, 10', 10", 110) can be connected to a connector on the tracheostomy cannula and the valve unit (20, 20', 20", 120) is arranged at the second end (12, 12', 112) of the attachment unit (10, 10', 10", 110), and wherein the valve unit (20, 20', 20", 120) and the attachment unit (10, 10', 10", 110) are connected together movably and preferably detachably,
**characterised in that**
the valve unit is designed and arranged in such a way that in a first position it closes the housing (13, 13', 13", 114) of the attachment unit (10, 10', 10", 110) air-tightly with respect to a flow direction, and
in at least one second position permits a flow of air into the housing (13, 13', 13", 114) and out of the housing (13, 13', 13", 114),
wherein the valve unit (20, 20', 20", 120) can be transferred from the first position into the at least one second position by a movement parallel to the direction of the longitudinal axis of the tubular housing (13, 13', 13", 114), wherein the valve unit (20, 20', 20", 120) has a disk-shaped apertured valve cover (24, 24', 24", 124) and an elastic valve diaphragm (25, 25', 125) which can be applied sealingly on the inside of the valve cover (24, 24', 24", 124) and which covers over the apertures (27, 27', 128) and lifts off a valve seat at the inside of the valve cover (24, 24', 24", 124) at least when a predeterminable pressure difference between the outside and the inside of the valve unit (20, 20', 20", 120) is exceeded.

2. The speaking valve (1, 1', 1", 101) as set forth in claim 1 **characterised in that** the valve unit (20, 20', 20", 120) comprises at least one lateral opening, which in the first position is covered by a wall of the attachment unit (10, 10', 10", 110), which bears against the surroundings of the lateral opening, and which in a second position is opened by the wall of the attachment unit.

3. The speaking valve (1, 1', 1", 101) as set forth in claim 1 or 2 **characterised in that** the attachment unit (10, 10', 10", 110) has at least one first connecting element (14, 14') and the valve unit (20, 20', 20", 120) has at least one second connecting element (26, 26'), wherein either the first connecting element (14, 14') or the second connecting element (26, 26') has a projection and the respective other connecting element has a receiving opening corresponding thereto, wherein the attachment unit (10, 10', 10", 110) and the valve unit are movably connected together by way of the mutually engaging first (14, 14') and second connecting elements (26, 26').

4. The speaking valve (1, 1', 1", 101) as set forth in claim 3 **characterised in that** the at least one projection and the at least one receiving opening are respectively formed by a screwthread or by a latching connection having at least two latching steps or a combination of thread and latching connection.

5. The speaking valve (1, 1', 1", 101) as set forth in one of claims 2 to 4 **characterised in that** the connecting elements have an inner cylinder and a hollow outer cylinder which engage into each other in concentric and fitting relationship and of which one is respectively fixedly connected to the valve unit and the other to the attachment unit (10, 10', 10", 110).

6. The speaking valve (1, 1', 1", 101) as set forth in claim 5 **characterised in that** the cylinder and the hollow cylinder are each of a smaller diameter than the lumen of the tubular attachment unit (10, 10', 10", 110) and are respectively arranged centrally with respect to the attachment unit and the valve unit.

7. The speaking valve (1, 1', 1", 101) as set forth in claim 5 **characterised in that** the cylinder in turn is a hollow cylinder and the cylinder and the hollow cylinder are formed by the tubular housing (13, 13', 13", 114) of the attachment unit (10, 10', 10", 110) and a cylindrical casing portion of a cap-shaped valve unit (20, 20', 20", 120).

8. The speaking valve (1, 1', 1", 101) as set forth in one of the preceding claims **characterised in that** an air-permeable filter element (16, 16', 105) is arranged in the flow cross-section of the second end (12, 12', 112) of the tubular housing (13, 13', 13", 114) of the attachment unit, preferably a filter element (16, 16', 105) which has a material for storing heat and/or moisture, in particular a hygroscopic material.

9. The speaking valve (1, 1', 1", 101) as set forth in one of the preceding claims **characterised in that** the valve diaphragm (25, 25', 125) has edge regions and a central region, wherein the central region is fixed to the valve cover (24, 24', 24", 124) in such a way that the edge regions of the diaphragm can lift elastically off the valve seat in the direction of the attachment unit under the action of a corresponding pressure force.

10. The speaking valve (1, 1', 1", 101) as set forth in claim 9 **characterised in that** the valve cover (24, 24', 24", 124) is also formed from an elastic material and in the first position bears with a prestressing against the second end (12, 12', 112) of the housing (13, 13', 13", 114) and in the second position opens a passage gap relative to the upper edge of the second end (12, 12', 112) of the tubular housing (13, 13', 13", 114) of the attachment unit.

11. The speaking valve (1, 1', 1", 101) as set forth in one of the preceding claims **characterised in that** the valve unit (20, 20', 20", 120) is in the form of a valve cap having a cylindrical cap casing portion and a disk-shaped valve cover (24, 24', 24", 124), wherein preferably the cap casing portion embraces at least the second end (12, 12', 112) of the tubular housing (13, 13', 13", 114) from the outside, wherein a sealing surface is provided on the inside of the valve cover (24, 24', 24", 124) or at the transition between the cap casing portion and the valve cover (24, 24', 24", 124), which sealing surface in the first position is in sealing engagement with the upper edge of the second end (12, 12', 112) of the tubular housing (13, 13', 13", 114).

12. The speaking valve (1, 1', 1", 101) as set forth in one of the preceding claims **characterised in that** the upper edge of the second end (12, 12', 112) of the tubular housing (13, 13', 13", 114) is in the form of a sealing lip.

13. The speaking valve (1, 1', 1", 101) as set forth in one of the preceding claims **characterised in that** the peripheral surface (23, 23') of the valve unit (20, 20', 20", 120) has a grooving, a roughening and/or a projection or a recess in the peripheral surface (23, 23') and/or the valve unit (20, 20', 20", 120) has one or more markings on its outwardly facing side.

14. The speaking valve (1, 1', 1", 101) as set forth in one of the preceding claims **characterised in that** provided between the valve unit (20, 20', 20", 120) and the attachment unit (10, 10', 10", 110) for at least one of the first and second positions are latching elements which audibly latch together.

15. The speaking valve (1, 1', 1", 101) as set forth in one of the preceding claims wherein the housing of the attachment unit (10, 10', 10", 110) and the peripheral surface (23, 23') of the valve unit (20, 20', 20", 120) are together of a conically outwardly tapering shape and/or the first end (11, 11', 111) of the attachment unit (10, 10', 10", 110) can be fitted on to the connector in frictionally locking relationship.

16. A tracheostomy cannula (30', 30") having a speaking valve (1, 1', 1", 101) as set forth in one of the preceding claims.

## Revendications

1. Valve de phonation (1, 1', 1", 101) destinée à être enfichée sur une canule de trachéotomie, qui comprend une unité de valve (20, 20', 20", 120) et une unité d'enfichage (10, 10', 10", 110), l'unité d'enfichage comportant corps tubulaire (13, 13', 13", 114) avec une première (11, 11', 111) et une seconde (12, 12', 112) extrémités et avec un axe longitudinal allant de la première (11, 11', 111) à la seconde (12, 12', 112) extrémité,
la première extrémité (11, 11', 111) de l'unité d'enfichage (10, 10', 10", 110) étant adaptée pour pouvoir être connectée à la canule de trachéotomie par un connecteur et l'unité de valve (20, 20', 20", 120) étant disposée à la seconde extrémité (12, 12', 112) de l'unité d'enfichage (10, 10', 10", 110) et l'unité de valve (20, 20', 20", 120) et l'unité d'enfichage (10, 10', 10", 110) étant mobiles et attachées l'une à l'autre de préférence de façon amovible, **caractérisée en ce que**
l'unité de valve est configurée et disposée de façon telle que,
dans une première position, elle ferme le corps (13, 13', 13", 114) de l'unité d'enfichage (10, 10', 10", 110) hermétiquement par rapport à une direction de flux et,
dans au moins une deuxième position, elle permet un flux d'air entrant dans le corps (13, 13', 13", 114) et sortant de celui-ci, l'unité de valve (20, 20', 20", 120) étant adaptée pour pouvoir être déplacé de la première position à ladite au moins une deuxième position par un mouvement parallèle à la direction de l'axe longitudinal du corps tubulaire (13, 13', 13", 114), l'unité de valve (20, 20', 20", 120) comprenant un couvercle de valve (24, 24', 24", 124) ayant la forme d'un disque et étant pourvu d'ajours et une membrane de valve élastique (25, 25', 125) adaptée pour pouvoir épouser de façon étanche la face intérieure du couvercle de valve (24, 24', 24", 124) et qui recouvre les ajours (27, 27', 128) et qui se soulève d'un siège de valve sur la face intérieure du couvercle de valve (24, 24', 24", 124) au moins lorsqu'une différence de pression pouvant être prédéterminée entre le côté extérieur et le côté intérieur de l'unité de valve (20, 20', 20", 120) est dépassée.

2. Valve de phonation (1, 1', 1", 101) selon la revendication 1, **caractérisée en ce que** l'unité de valve (20, 20', 20", 120) comporte au moins un évidement qui, dans la première position, est recouvert par une paroi de l'unité d'enfichage (10, 10', 10", 110) accolée aux alentours de l'évidement, et qui, dans la deuxième position, est rendu libre par la paroi de l'unité d'enfichage.

3. Valve de phonation (1, 1', 1", 101) selon la revendication 1 ou 2, **caractérisée en ce que** l'unité d'enfichage (10, 10', 10", 110) comprend au moins un premier élément de connexion (14, 14') et que l'unité de valve (20, 20', 20", 120) comprend au moins un deuxième élément de connexion (26, 26'), soit le premier (14, 14') soit le deuxième (26, 26') élément de connexion ayant une avancée et le respectivement autre élément de connexion ayant un évidement de réception correspondant à celle-ci, l'unité d'enfichage (10, 10', 10", 110) et l'unité de valve étant reliées l'une à l'autre de manière mobile par les premiers (14, 14') et les deuxièmes (26, 26') éléments de connexion.

4. Valve de phonation (1, 1', 1", 101) selon la revendication 3, **caractérisée en ce que** ladite au moins une avancée et ledit au moins un évidement de réception sont formés chacun par une liaison à filetage ou par une liaison à encliquetage avec au moins deux crans d'encliquetage ou par une combinaison de filetage et de liaison à encliquetage.

5. Valve de phonation (1, 1', 1", 101) selon l'une des revendications 2 à 4, **caractérisée en ce que** les éléments de connexion comprennent un cylindre intérieur et un cylindre extérieur creux qui s'emboîtent de manière concentrique et à taille correcte, et dont l'un est solidaire de l'unité de valve et dont l'autre est solidaire de l'unité d'enfichage (10, 10', 10", 110).

6. Valve de phonation (1, 1', 1", 101) selon la revendication 5, **caractérisée en ce que** le cylindre et le cylindre creux présentent chacun un diamètre inférieur à la lumière de l'unité d'enfichage (10, 10', 10", 110) en forme de tuyau et qu'ils sont disposés chacun de manière centrée par rapport à l'unité d'enfichage et l'unité de valve.

7. Valve de phonation (1, 1', 1", 101) selon la revendication 5, **caractérisée en ce que** le cylindre est lui-même un cylindre creux et **en ce que** le cylindre et le cylindre creux sont formés par le boîtier en forme de tuyau (13, 13', 13", 114) de l'unité d'enfichage (10, 10', 10", 110) et un manchon cylindrique d'une unité de valve (20, 20', 20", 120) en forme de capuchon.

8. Valve de phonation (1, 1', 1", 101) selon l'une des revendications précédentes, **caractérisée en ce qu'**un élément filtrant (16, 16', 105) perméable à l'air, de préférence un élément filtrant (16, 16', 105) qui comprend un matériau emmagasinant de la chaleur et/ou de l'humidité, notamment un matériau hygroscopique, est disposé dans la section transversale d'écoulement de la deuxième extrémité (12, 12', 112) du boîtier en forme de tuyau (13, 13', 13", 114) de l'unité d'enfichage.

9. Valve de phonation (1, 1', 1", 101) selon l'une des revendications précédentes, **caractérisée en ce que** la membrane de valve (25, 25', 125) comprend des zones de bordure et une zone centrale, la zone centrale étant fixée au couvercle de valve (24, 24', 24", 124) de manière telle que les zones de bordure de la membrane puissent se soulever élastiquement du siège de valve dans la direction de l'unité d'enfichage sous l'influence d'une force de pression correspondante.

10. Valve de phonation (1, 1', 1", 101) selon la revendication 9, **caractérisée en ce que** le couvercle de valve (24, 24', 24", 124) est également réalisé en un matériau élastique et que, dans la première position, il est hermétiquement en appui, sous une précontrainte, sur la deuxième extrémité (12, 12', 112) du boîtier (13, 13', 13", 114) et que, dans la deuxième position, il laisse libre une fente de passage vers le bord supérieur de la deuxième extrémité (12, 12', 112) du boîtier en forme de tuyau (13, 13', 13", 114) de l'unité d'enfichage.

11. Valve de phonation (1, 1', 1", 101) selon l'une des revendications précédentes, **caractérisée en ce que** l'unité de valve (20, 20', 20", 120) est réalisée sous la forme d'un capuchon de valve avec un manchon cylindrique de capuchon et un couvercle de valve (24, 24', 24", 124) en forme de disque, de préférence, le manchon de valve entourant à l'extérieur au moins la deuxième extrémité (12, 12', 112) du boîtier en forme de tuyau (13, 13', 13", 114), une surface d'étanchéité étant prévue sur la face intérieure du couvercle de valve (24, 24', 24", 124) ou à la transition entre le manchon de capuchon et le couvercle de valve (24, 24', 24", 124), laquelle surface est en engagement d'étanchéité, dans la première position, avec le bord supérieur de la deuxième extrémité (12, 12', 112) du boîtier en forme de tuyau (13, 13', 13", 114).

12. Valve de phonation (1, 1', 1", 101) selon l'une des revendications précédentes, **caractérisée en ce que** le bord supérieur de la deuxième extrémité (12, 12', 112) du boîtier en forme de tuyau (13, 13', 13", 114) est réalisé comme lèvre d'étanchéité.

13. Valve de phonation (1, 1', 1", 101) selon l'une des revendications précédentes, **caractérisée en ce que** la surface circonférentielle (23, 23') de l'unité de valve (20, 20', 20", 120) comporte un rainurage, une rugosité et/ou une excroissance ou un enfoncement dans la surface circonférentielle (23, 23') et/ou que l'unité de valve (20, 20', 20", 120) comporte, sur son côté orienté vers l'extérieur, un ou plusieurs marquages.

14. Valve de phonation (1, 1', 1", 101) selon l'une des revendications précédentes, **caractérisée en ce que** sont prévus, entre l'unité de valve (20, 20', 20", 120) et l'unité d'enfichage (10, 10', 10", 110), pour au moins une des premières et deuxièmes positions, des éléments d'encliquetage qui s'engagent les uns dans les autres de manière audible.

15. Valve de phonation (1, 1', 1", 101) selon l'une des revendications précédentes, le boîtier de l'unité d'enfichage (10, 10', 10", 110) et la surface circonférentielle (23, 23') de l'unité de valve (20, 20', 20", 120) ayant ensemble une forme s'amenuisant vers l'extérieur de manière conique et/ou la première extrémité (11, 11', 111) de l'unité d'enfichage (10, 10', 10", 110) étant enfichable sur le connecteur de façon solidaire en friction.

16. Canule de trachéotomie (30', 30") avec une valve de phonation (1, 1', 1", 101) selon l'une des revendications précédentes.
